# NEW EUROPEAN PATENT SPECIFICATION

(11) **EP 0 148 466 B2**
(45) Date of publication and mention of the opposition decision: **26.06.2002**
(45) Mention of the grant of the patent: 07.03.1990
(21) Application number: 84115691.2
(22) Date of filing: 18.12.1984
(51) Int. Cl.: A61K 7/13

(54) **Hair dye compositions**
Haarfärbemittel
Compositions pour teinture des cheveux

(30) Priority: 23.12.1983 JP 24349383
(43) Date of publication of application: 17.07.1985
(73) Proprietor: Kao Corporation, Chuo-Ku Tokyo 103 (JP)
(72) Inventor: Nomura, Tadashi, Sakura-shi, Chiba-ken (JP); Ikeda, Yasuhiro, Sakura-shi, Chiba-ken (JP); Nemoto, Toshiyuki, Hoya-shi, Tokyo (JP)
(74) Representative: Wächtershäuser, Günter, Prof. Dr.

(56) References cited:
- DD-A- 10 140
- DE-A- 2 028 818
- DE-A- 2 307 596
- FR-A- 1 365 276
- US-A- 3 857 674
- Nowak, G.A: et al. : "Die kosmetischen Präparate. Rezeptur, Herstellung uns wissenschaftliche Grundlagen" 2. verbesserte und erweiterte Auflage, Verlag für chemische Industrie H. Zlolkowsky KG, Augsburg (1975)pages 610-611
- Preisinger, S.: "Das Haarfärben und Aufhellen mit KLEINOL Gelee" (1952) pages 5-9 and 60-65
- Fey, H.: "Wörterbuch der Kosmetik" Wissenschaftliche Verlagsgesellschaft MBH, Stuttgart (1974) pages 430-431
- Bates, R.G.: "Determination of pH. Theory and Practice" John Wiley & Sons (1964) page 319, tables 5-11

## Description

### i) Field of the Invention

This invention relates to a hair dye composition, and more particularly to a bi-liquid oxidation type hair dye composition which comprises a specified salt and ammonia, thereby achieves decoloration or hair dye in a deep color tone at a comparatively low pH level.

### ii) Description of the Prior Art

In a broad sense, so-called hair dye compositions include "a hair dye composition" for dying hair and "a bleach composition" for decoloring hair. In this specification, hair dye compositions include both categories. These hair dye compositions are used for the purpose of beauty treatment, and include so-called "white-hair dye compositions" to dye white hair to a desired color such as black, black-brown or blonde so called "fashion dye composition" to dye hair to a brighter color and so-called "white-hair/fashion dye compositions" to cover both functions.

These dye compositions are required to color whole hair or a part of hair to be dyed completely and securely, and to give a natural feeling.

The hair dye compositions used for such purposes include oxidation type permanent hair dye compositions and non-oxidation type semi-permanet hair dye compositions or temporary hair dye compositions (hair colors). Among which, bi-liquid oxidation type permanent hair dye compositions which comprise the color lotion containing dye-intermediates and an alkali agent, and the oxidizer containing an oxidant, are the most popular, since they offer more plentiful color tones than those of other type hair dyes, and also satisfy the above-mentioned requirements.

A bi-liquid oxidation type hair dye composition decomposes compounds such as peroxide of an oxidant with an alkali agent to generate oxygen, which makes decomposition/decoloration of melanine, and produces the oxidized dye by oxidation/polymerization of dye-intermediates, resulting in hair dying. Therefore, if the oxidation/polymerization and the melanine decomposition are insufficient, the dyed hair shows no deep and natural tone with incongruity. These two reactions are strongly dependent on pH values. Since no satisfactory result is obtainable at acidic and neutral pH levels, the pH value is generally fixed at 10 or more.

However, hair treatment at a high pH level damages hair and skin considerably, while a lower pH value makes the dying effect inferior. Thus, both functions have not been simultaneously satisfied.

### Summary of the Invention

Under such circumstances, the inventors have studied to solve the above defects and found that use of ammonia as an alkali agent with a water soluble ammonium salt shows an excellent dyeing effect even at a low pH level, resulting in the production of a new hair dye composition without damage to hair or skin.

Accordingly this invention provides a bi-liquid oxidation type hair dye composition as defined in claim 1, and a hair bleach composition as defined in claim 2.

### Detailed Description of the Invention and Preferred Embodiments

Water soluble ammonium salts usable in this invention are ammonium chloride and ammonium nitrate, which are preferable since they have good dyeability. Other ammonium salts are rather inferior to those salts in view of dyeability and have some possibility to occur exothermic problem. Such a watersoluble ammonium salt is incorporated in the color lotion in an amount to adjust its content in the hair dye mixture to 1 to 5 weight percent, when both of the color lotion and the oxidizer are mixed at the time of use. No satisfactory effect appears at less than 1 weight percent, and no better result is also obtainable at a higher level than 5 weight percent.

The quantity of ammonia incorporated in the color lotion is adjusted so as to keep the pH of above hair dye composition mixture at 7 to 9.5.

In addition, the conventional oxidation type hair dye bases are mixed in the color lotion and in the oxidiser. The oxidation type hair dye bases include dye-intermediates, oxidants and also reactive compounds as coupler (modifiers) used together as occasion demands. Generally used dye-intermediates include para and other forms of such compounds as p-phenylenediamine, toluene-2,5-diamine, N-phenylp-phenylenediamine, 4,4'-dimaniodiphenylamine, p-aminophenol, p-methylaminophenol, o-phenylenediamine, toluene-3, 4-diamine, o-aminophenol, p-chloro-o-phenylene diamine, p-amino-o-cresol, o-chlorp-phenylenediamine, phloroglucinol, pyrogallol, 3,3'-iminodiphenyl, diphenylamine, 2,6-diaminophridine and p-aminophenylsulfamic acid. As couplers (modifiers), phenols and meta forms of such compounds as m-phenylenediamine, , toluene-2, 4-diamine, p-methoxy-m-phenylenediamine, m-aminophenol, alpha-naphthol, resorcinol, hydroquinone and cathecol, are generally used. Among these couplers, in the case of black, chestnut, brown or blonde dyeing, p-phenylenediamine, or toluene-2,5-diamine is especially important. Also, as oxidants, hydrogen peroxide, sodium perborate, urea peroxide, sodium percarbonate, sodium tripolyphosphate peroxide, sodium pyrophosphate peroxide, sodium orthophosphate peroxide, sodium silicate peroxide additive and sodium sulfate sodium chloride peroxide additive, are used. In addition to the above compounds, the following materials as dyes which influence the hair color tones without direct relation to the reaction of color formation can be added in order to keep the quality of hair dye and enhance its usefulness; for example, nitro-dyes such as nitro-p-phenylenediamine, p-nitro-o-phenylenediamine, 2-amino-4-nitrophenol, 2-amino-5-nitrophenol and 4-amino-2-nitro phenol, and direct dyes such as picramic acid, picric acid and 1,4-diaminoanthraquinone; surfactants such as nonion surfactants, anion surfactants, cation surfactants and ampholite surfactants; solvents such as propylene glycol, glycerin and urea; lower alcohols such as ethyl alcohol and isopropyl alcohol; viscosity compensators such as hydroxy ethylcellulose, methyl cellulose, cationic high polymer compounds and higher alcohols; colours, sun-screen agents, antioxidants, preservatives, pearl agents, lotion agents, stabilizers, osmotic agents, moisturizers, haircare agents, perfume oil, vaselin, liquid parafin and other materials.

### Example 1

Bi-liquid type bleach compositions with the following formulations were prepared, and degrees of decoloration and degrees of smooth combing (degrees of hair damage) were determined.

### (1) Bleach Compositions

Color lotions: as shown in Table 1.

Oxidizers: peroxide 6% phosphoric acid adjusted at pH 4: purified water: balance.

**TABLE 1**

| Ingredients (%) | Control | Product of This Invention | | | | |
|---|---|---|---|---|---|---|
| | A | B | C | D | E | F |
| Ammonium nitrate | ― | 3 | 5 | 10 | 1 5 | ― |
| | | | | | | |
| Ammonium chloride | ― | ― | ― | ― | ― | 3 |
| | | | | | | |
| Oleic acid | 10 | 10 | 10 | 10 | 10 | 10 |
| | | | | | | |
| Polyoxythylene (10) | oleyl ether | 6 | 6 | 6 | 6 | 6 |
| | | | | | | |
| Polyoxythylene (2) | oleyl ether | 4 | 4 | 4 | 4 | 4 |
| | | | | | | |
| Propylene glycol | 10 | 10 | 10 | 10 | 10 | 10 |
| | | | | | | |
| Perfume oil | 0.3 | 0.3 | 0.3 | 0.3 | 0.3 | 0.3 |
| | | | | | | |
| Aqueous ammonia | Adjusted pH to 9 | " | " | " | " | " |
| | | | | | | |
| Purified water | Balance | " | " | " | " | " |

### (2) Treatment Procedure

Black virgin hair of 20 cm long was washed with 0.5% lauryl sodium sulfate aqueous solution, and air-dried to prepare the test hair. Twenty grams of the test hair was bundled, and spread with the same volume mixture of a color lotion and an oxidiser (pH 8.8) as a bleach at the bath ratio of 1:1, and allowed to stand for 30 min. The treated hair was rinsed with running water at 40°C, and washed with 0.5% of lauryl sodium sulfate aqueous solution followed by again rinsing with running water and air-drying.

### (3) Determination of Decoloration Degrees

The brightness of hair after the decoloration treatment was determined with Colorimeter Computer ND-1010C (Nippon Denshoku Kogyo K. K.). The L valves in Table 2 indicate the brightness degrees, and higher values mean brighter degrees of decolored hair.

### (4) Combining Force

The bundle of decolored hair was combed with a comb fixed at the movable part of a tension tester (Tokyo Sokki UTM-II type), and the tension loaded on the hair bundle was determined as the combing force. The combing force in Table 2 are average values and deviations for 20 times of combing.

### (5) Results

**TABLE 2**

| Assay | Control | Products of This Invention | | | | |
|---|---|---|---|---|---|---|
| | A | B | C | D | E | F |
| Decoloration degree (L value) | 24.8 | 43.8 | 44.5 | 45.8 | 45.5 | 44.0 |
| | | | | | | |
| Combing force (g) | 240 ±50 | 170 ±20 | 168 ±20 | 170 ±30 | 185 ±50 | 172 ±30 |

### Example 2

Bi-liquid oxidation type hair dye compositions of the following formulations were prepared for dying hair, and the dying degrees of white human hair and the combing forces were determined.

### (1) Hair Dye Compositions

Color lotions: as shown in Table 3.

**TABLE 3**

| Ingredients (%) | Control | Products of This Invention | |
|---|---|---|---|
| | L | M | N |
| p-Phenylenediamine | 2 | 2 | 2 |
| | | | |
| o-Aminophenol | 1 | 1 | 1 |
| | | | |
| m-Phenylenediamine | 0.2 | 0.2 | 0.2 |
| | | | |
| Ammonium nitrate | ― | 3 | ― |
| | | | |
| Ammonium chloride | ― | ― | 3 |
| | | | |
| Oleic acid | 10 | 10 | 10 |
| | | | |
| Polyethylene (10) oleylether | 4.5 | 4.5 | 4.5 |
| | | | |
| Polyoxyethlene (2) oleylether | 4.5 | 4.5 | 4.5 |
| | | | |
| Propylene glycol | 15 | 15 | 15 |
| | | | |
| Thiglycolic acid | 0.5 | 0.5 | 0.5 |
| | | | |
| Sodium edetate | 2 | 2 | 2 |
| | | | |
| Perfume oil | 0.3 | 0.3 | 0.3 |
| | | | |
| Aqueous ammonia ammonia | Adjusted pH to 9 | " | " |
| | | | |
| Purified water | Balance | " | " |

Oxidizers: peroxide 6% phosphoric acid adjusted at pH 9; purified water; balance

### (2) Hair Treatment Procedure

Same as (2) of Example 1 except the test hair, for which white human hair of 20 cm long was used.

### (3) Determination of Dying Degrees

Same as (3) of Case 1, but lower L values in Table 4 indicate darker and deeper colors.

### (4) Determination of Combing Forces

Same as (4) as Example 1.

### (5) Results

**TABLE 4**

| | Control | Products of This Invention | |
|---|---|---|---|
| Assay | L | M | N |
| Dyeing degree (L value) | 23.2 | 13.1 | 14.0 |
| | | | |
| Combing force (g) | 310 ±50 | 200 ±20 | 200 ±40 |

### Example 3

Hair was dried with the bi-liquid oxidation type hair dyes used in (1) of Example 2, and the color tones and touch feels of dyed hair were evaluated by a panel of 20 women.

### (1) Hair Treatment Procedure

Same as (2) of Example 2.

### (2) Evaluation of Color Tones of Dyed Hair

A panel of twenty women evaluated the darkness degree comparing a test hair and the control both of which were dyed with hair dyes in Table 5.

### (3) Evaluation of Touch Feels of Dyed Hair

A panel of twenty women evaluated the following items comparing a test hair with the control.
1) Softness
2) Smoothness
3) Combing condition

### (4) Results

**TABLE 5**

| Items of Evaluation | Products of This Invention | |
|---|---|---|
| | M | N |
| Darkness | 18 | 16 |
| | | |
| Softness | 17 | 17 |
| | | |
| Smoothness | 18 | 17 |
| | | |
| Good combing | 18 | 16 |

The above figures indicate the number of evaluators who elect a product of this invention (M or N) as superior to the control L.

* The evaluation was made at N=20.

## Claims

1. A bi-liquid type hair dye composition comprising (1) a color lotion component and (2) an oxidizer component,
said color lotion component comprising (a) a dye intermediate and coupler in amounts effective for dyeing of hair, (b) ammonia in an amount effective to provide the composition with a pH of 7 to 9.5, and (c) ammonium chloride or ammonium nitrate in an amount effective to provide the composition with a concentration of 1 to 5 weight %, and effective for increasing the dyeing degree of the dye chosen,
said oxidizer component comprising an oxidizing agent in an amount effective for dyeing of hair.

2. A bi-liquid type hair bleach composition comprising (1) a color lotion component and (2) an oxidizer component,
said color lotion component comprising (a) ammonia in an amount effective to provide the composition with a pH of 7 to 9.5, and (b) ammonium chloride or ammonium nitrate in an amount effective to provide the composition with a concentration of 1 to 5 weight %, and
said oxidizer component comprising an oxidizing agent in an amount effective for decoloring of hair.

## Patentansprüche

1. Zusammenstellung zum Haarefärben aus zwei Flüssigkeiten, umfassend (1) eine Färbelotionskomponente und (2) eine Oxidationskomponente, wobei die Färbelotionskomponente enthält:
(a) ein Farbstoffintermediat und ein Kopplungsmittel in zum Haarefärben wirkungsvollen Mengen,
(b) Ammoniak in einer Menge, um die Zusammensetzung auf einen pH-Wert von 7 bis 9,5 einzustellen und
(c) Ammoniumchlorid oder Ammoniumnitrat in einer Menge, die der Zusammensetzung eine Konzentration von 1 bis 5 Gewichts-% verleiht und die zur Steigerung des Färbegrades des gewählten Farbstoffes wirkungsvoll ist,
und wobei die Oxidationskomponente ein Oxidationsmittel in einer zum Haarefärben wirkungsvollen Menge enthält.

2. Zusammenstellung zum Haarebleichen aus zwei Flüssigkeiten, umfassend (1) eine Färbelotionskomponente und (2) eine Oxidationskomponente, wobei die Färbelotionskomponente enthält:
(a) Ammoniak in einer Menge, um die Zusammensetzung auf einen pH-Wert von 7 bis 9,5 einzustellen und
(b) Ammoniumchlorid oder Ammoniumnitrat in einer Menge, die der Zusammensetzung eine Konzentration von 1 bis 5 Gewichts-% verleiht, und wobei die Oxidationskomponente ein Oxidationsmittel in einer zum Entfärben von Haaren wirkungsvollen Menge enthält.

## Revendications

1. Composition pour la teinture des cheveux, du type à deux composants liquides, comprenant (1) un composant lotion colorante et (2) un composant agent d'oxydation,
ledit composant lotion colorante comprenant (a) un intermédiaire de teinture et un coupleur en des quantités efficaces pour teindre les cheveux, (b) de l'ammoniaque en une quantité efficace pour doter la composition d'un pH de 7 à 9,5, et (c) du chlorure d'ammonium ou du nitrate d'ammonium en une quantité efficace pour en doter la composition d'une concentration de 1 à 5 % en poids, et efficace pour augmenter le degré de coloration du colorant choisi,
ledit composant agent d'oxydation comprenant un agent d'oxydation en une quantité efficace pour teindre les cheveux.

2. Composition pour la décoloration des cheveux du type à deux composants liquides, comprenant (1) un composant lotion colorante et (2) un composant agent d'oxydation, ledit composant lotion colorante comprenant (a) de l'ammoniaque en une quantité efficace pour doter la composition d'un pH de 7 à 9,5, et (b) du chlorure d'ammonium ou du nitrate d'ammonium en une quantité efficace pour en doter la composition d'une concentration de 1 à 5 % en poids, et
ledit composant agent de coloration comprenant un agent d'oxydation en une quantité efficace pour décolorer les cheveux.
